# EUROPEAN PATENT APPLICATION

(11) **EP 3 144 269 A1**
(43) Date of publication of application: **22.03.2017**
(21) Application number: 15386013.5
(22) Date of filing: 27.05.2015
(51) Int. Cl.: B67D 7/32, B64F 1/28, G01N 30/68

(54) **MOBILE UNIT FOR ENABLING QUALITATIVE AND QUANTITATIVE FUEL CONTROL**

(30) Priority: 28.05.2014 GR 20140100298
(71) Applicant: Sunfield Shipping Limited, 2025 Strovolos (CY)
(72) Inventor: MAVRAKIS, Stylianos, 145 64 Nea Kifisia Attikis (GR); GRIGORAKIS, Emmanouil, 81 100 Mytilini Lesvos (GR)
(74) Representative: Papaconstantinou, Helen

(57) **Abstract**

Mobile unit to enable fuel qualitative and quantitative analyses e.g. in real time and comprising an expansion safety-valve (1) causing the fuel (A) to pass through a filter (2) with a first pump (3) injecting fuel residues (impurities such as sulphur, carbon and the like) in a residue tank (4) and a second pump (3") channeling fuel into a channel for quantitative (6, such as volume, flow-rate and other kinematics) and qualitative (5, such as mass, density, viscosity, temperature and flash-point) controls.

## Description

The System certifies the accurate recording of the quantity of fuel (marine, diesel, heating) filtered from each residue (supplying ships, industries, construction sites, premises) and a detailed description of the chemical composition of the fuel. Quality control certifies the elemental analysis, excluding the receiving of improper fuel with a forbidden content of harmful elements (sulfur, carbon) that are responsible for the greenhouse effect and acid rain, when at the oil combustion is converted into carbon dioxide (CO₂) and sulphuric acid (H₂SO₄) respectively.

Fuel collection is performed either by supply tankers ashore (maritime fuel, diesel fuel, heating oil) or with special supply ships of maritime fuel at sea which have a counter or counters of the supplied quantity. These counters are disadvantaged because they do not have a system for determining quality characteristics, secure system of quantitative recording and discharge fuel system from impurities and residues. In this method the disadvantage for the receiver lies in the fact that they are supplied with fuel without a qualitative analysis and quantitative control certification. The adulterated, improper fuel (in the distribution and supply), is fairly commonplace, with the presumed negative consequences for the environment of the private and national economy.

The present invention creates a mobile unit which, composed of independent organs of modem technology with a proper assembly and with a special filter, is interposed between the means (a tank vehicle or ship) of the fuel supply and the receiving tanks (8). It filters the fuel, clearing it of any residuals and captures with complete data the picture of its net quantity and quality. As an invention, is presented the combination of a series of specialized high-tech and technical tools with the appropriate assembly between them so as to achieve the above purpose.

The advantage of the invention is that the Mobile Unit System of Qualitative and Quantitative Fuel Control is interposed between each fuel supplier (A) and the tanks of the collector (B) and provides, in real-time, the most precise record of data of the received quantity, filtered from impurities and residues and secondly identifies the quality characteristics to prevent any adulteration and irregularity.

Scheme 1 depicts a floor plan of the Mobile Unit System of Qualitative and Quantitative Control.

Schemes 2 and 3 depict the way that the invention is implemented with the appropriate assembly of the application.

The fuel that originates from the supplier (A) shall follow the path below through the system. Expansion safety valve (1) that, in case of increased pressure, injects the necessary amount in the tank residues in order to
regularize the flow of the fuel. Filter (2) removes the inappropriate elements and, through the pump (3), injects them in the residue tank (4). Quality control organs (5) of the fuel that verify the identity and the unique features of each fuel type. Mass flow meter tool and tool for the fuel's quantitative control (6) suitable for measurements of 10,000 liters/hour up to 200.000 liters/hour. Fuel tanks of the collector (B) (ship, industry, manufacture, factory, construction site, building complex).

Initially, the fuel passes through the special filter (2), by inserting the expansion safety valve (1) in the event of increased pressure of the fuel's flow,which removes inappropriate items and which, through the pump (3), are channeled in the residue tank (4). Later, a fuel's sample is taken via the special spout located inside the lab space, where the tools of the quality inspection are also installed) (5). This sample is simultaneously introduced in the flash point Analyzer (flash point tester), in the sulphur Analyzer and the kinematic viscosity meter. At the same time, the density is controlled and recorded by the density flow meter and the quantity of fuel is controlled by the quantitative control tool (6) before arriving at the ship's tanks and any kind of facility. This verifies the identity of the fuel and its unique characteristics. This verifies the identity of the fuel and its unique characteristics. All the above can be sent in real time (real time) to the directly concerned. This way the receiver has a clear picture of the quality (in accordance with the legislation in force) and of the supplied fuel quantity, which is free of waste.

Based on the European and national legislation, the Mobile Unit System of Qualitative and Quantitative Control of the fuel provides reliable service, on the collection and the delivery of the fuel. This process is divided into three parts. A) Purification of the fuel from water and foreign particles, so that collectors of all forms of fuel, are fully secured. The residue obtained from filtering are collected in a collector tank (4). B) Accurate recording of the mass, weight and density. C) Qualitative laboratory analysis on basic parameters of fuel based on its specifications. I.e., sulphur content, kinematic viscosity and flash point.
Thus, the technological equipment of the Unit System of Qualitative and Quantitative Fuel Control includes: a) kinematic viscosity Control in accordance with ISO 3104, b) Sulphur Control in accordance with ISO 13032, ISO 20846, c) control of flashpoint (flash point), in accordance with ISO 2719. Please note, that this entire process, can also be transmitted via Internet, at the time measurements are being performed (real time).

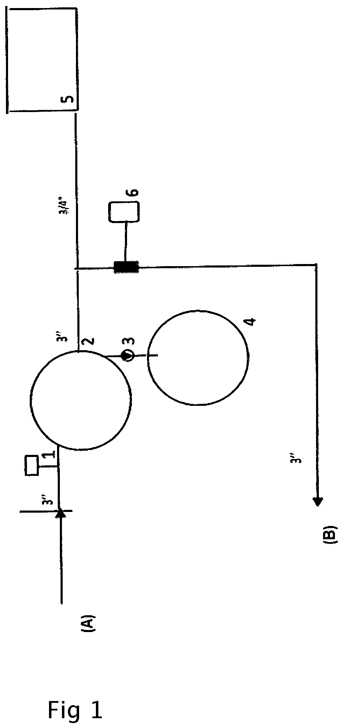

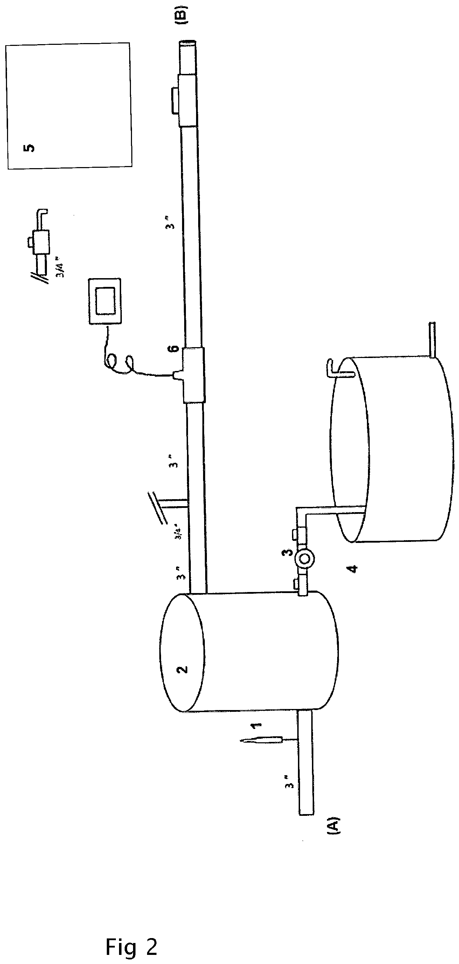

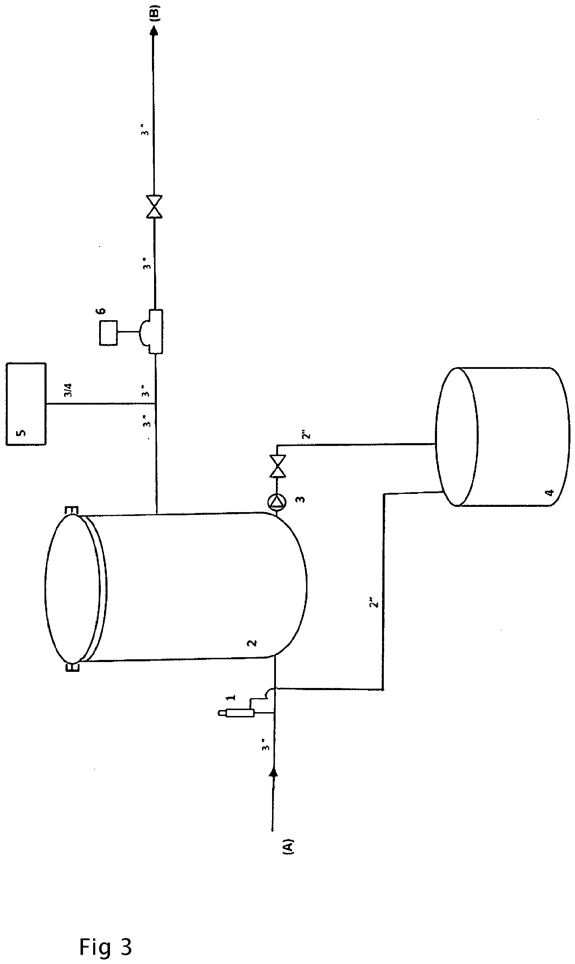

## Claims

1. The Mobile Unit System of Qualitative and Quantitative Fuel Control consists of the combination of technologically advanced tools, which with the attempted assembly between them characterize the creation of a control system for quantitative and qualitative identification of fuel (marine, diesel, heating), which is free from any impurity and residual and which is supplied with certification on ships, industries, factories, construction sites and premises. In particular, it consists of the following:
Expansion safety valve (1) which, in the event of
increased pressure when supplying the system with fuel, discharges the required amount of fuel in the residue tank of the system, filter with ventilation (2) which rejects the inappropriate elements, contained in the fuel, in the residue tank, tool of the fuel's qualitative analysis (5), which allows the simultaneous recording of various variables (mass , density, temperature).
They are extremely precise and operate independently of the physical properties of the fluid (viscosity, density, etc.).They are in position to measure 10,000 to 200,000 liters per hour, by changing the flow meter type at temperature and fluid pressure up to 350 C and 350 bar respectively, and flow meter of density and quantity of fuel (6) which is adjusted with the previous device, and reads the relevant information (fuel quantity, density, temperature), tank residues (4), where end up the fuel resulting from the expansion and the irregular elements that may be present in the fuel to be measured ,a pump (3) to transfer fuel and residues in the tank, a link for the delivery of the residues and various parts (valves, pipes etc.) for the proper assembly and operation of the network.
The fuel at the time of the delivery (A) passes through the special filter (2), by inserting the expansion safety valve (1) in the event of increased pressure of the fuel flow, which removes inappropriate elements and which, through the pump (3), are collected in the residue tank (4). Then, a fuel's sample is taken from the special spout located inside the lab space, where the tools
of the quality control are also installed (5). This sample is simultaneously introduced in the flash point Analyzer (flash point tester), in the sulphur Analyzer and the kinematic viscosity meter. At the same time, the density is controlled and recorded by the density flow meter and the quantity of the passing fuel is controlled by the quantitative control tool (6) before it ends up to the receiver (b).

2. Mobile Unit System of Qualitative and Quantitative Fuel Control according to the requirement 1 is **characterized by** the fact that it is interposed between the supplier (A) (any kind of tanker vehicle ashore, any kind of special supply ships of maritime fuel at the sea ) and the receiver (B) (ship, industry, manufacture, factory, construction site, building complex).
